# FASCICULE DE BREVET EUROPEEN

(11) **EP 0 424 292 B1**
(45) Date de publication et mention de la délivrance du brevet: **19.07.1995**
(21) Numéro de dépôt: 90420450.0
(22) Date de dépôt: 17.10.1990
(51) Int. Cl.: A61B 17/60

(54) **Dispositif externe de contention osseuse**
Vorrichtung zur externen Knochenfixierung
External device for bone immobilisation

(30) Priorité: 17.10.1989 FR 8913877
(43) Date de publication de la demande: 24.04.1991
(73) Titulaire: FIXANO SA, F-01000 Bourg en Bresse (FR)
(72) Inventeur: Gentil, Pierre, F-01000 Bourg-En-Bresse (FR); Martin, Jean-Jacques, F-69730 Genay (FR)
(74) Mandataire: Maureau, Philippe

(56) Documents cités:
- EP-A- 0 024 256
- EP-A- 0 099 289
- EP-A- 0 146 872
- US-A- 4 475 546

## Description

La présente invention a pour objet un dispositif de contention osseuse, plus spécialement destiné à des applications de traumatologie osseuse, telles que réduction de fractures, ou à des applications orthopédiques telles qu'allongement osseux, arthrodèse, ostéotomie, correction angulaire, etc....

Après fracture d'un os, il est nécessaire de procéder à la réduction de la fracture, puis à l'immobilisation du membre considéré pendant un certain temps permettant la reconstitution osseuse. Le moyen le plus traditionnel pour réaliser une telle immobilisation consiste à envelopper le membre contenant l'os à réparer par un plâtre.

Toutefois, la solution du plâtre n'est pas toujours possible, notamment lorsqu'il existe des plaies. Il est donc connu de réaliser, au cours d'une opération d'une part la réduction et d'autre part la mise en place de broches et de plaques en vue d'immobiliser l'os brisé dans sa position de consolidation. Cette technique, si elle donne de bons résultats, nécessite toutefois, après consolidation, une nouvelle intervention chirurgicale pour retirer les plaques et vis. En outre, une telle solution ne permet pas, si le réalignement des tronçons d'os n'est pas parfait dès l'origine, d'effectuer la correction adaptée.

Une technique de contention osseuse externe a été développée depuis quelques années par ILIZAROV. Cette technique consiste à engager dans chaque tronçon d'os des broches ou vis orientées sensiblement transversalement à l'os. Chaque série de broches est fixée sur un anneau entourant le membre considéré, les deux anneaux ou les différentes séries d'anneaux étant reliées les unes aux autres par des tiges fixées de façon réglable sur les anneaux. Cette solution est moins traumatisante que celle consistant à visser des plaques au contact direct de l'os, puisque les broches peuvent être mises en place sous anesthésie locale, et qu'en fin de reconstitution osseuse, elles sont retirées sans douleur pour le patient sans nécessiter une intervention chirurgicale. D'autre part, il est possible, en cours de traitement, de modifier le réglage de l'appareil. Cet appareil peut convenir tout aussi bien pour des consolidations de fractures, que pour des applications orthopédiques telles qu'allongement osseux, arthrodèses, ostéotomie, ou correction angulaire.

Les anneaux entourant le membre à traiter étant très encombrants, il a été imaginé de ne disposer que d'une seule tige latérale sur laquelle sont fixés les éléments portant les broches engagées dans les tronçons d'os.

Il existe différents types de dispositifs de contention externe, adaptés aux différentes applications, étant précisé que dans certaines applications, il est avantageux que les supports de broches soient liés rigidement l'un à l'autre, tandis que dans d'autres applications mettant en oeuvre notamment une reconstitution osseuse, il est avantageux que les deux supports de broches reliés l'un à l'autre puissent bénéficier d'un certain jeu élastique qui favorise la formation de l'os.

La demande de brevet européen N° 0 099 289 décrit un dispositif externe de contention osseuse comprenant deux mâchoires destinées chacune à la fixation de plusieurs vis ou similaires engagées dans l'une des deux parties de l'os dont la contention est à réaliser, ces deux mâchoires étant montées sur un même support s'étendant parallèlement au membre à équiper. Ce support comprend un corps en forme de cylindre d'axe sensiblement parallèle au membre à équiper, dans lequel sont montés deux pistons dont chacun est solidaire d'une tige destinée à la fixation de l'une des deux mâchoires. L'un des deux pistons est associé à des moyens de réglage de sa position axiale dans le cylindre et à des moyens de blocage dans celui-ci, tandis que l'autre piston est associé à des moyens élastiques tendant à le pousser vers l'extérieur, et à des moyens de blocage dans le cylindre.

Le but de l'invention est de fournir un dispositif externe de contention osseuse, qui permette tout aussi bien d'associer de façon rigide les deux pièces support de broches, que de les associer à l'aide d'un montage légèrement élastique, et qui soit simple à fabriquer et à utiliser tout en remplissant parfaitement sa fonction.

A cet effet, dans ce dispositif, du type précité, le piston équipé de moyens de réglage axial est monté bloqué en rotation dans le cylindre mais libre en translation dans celui-ci, et est solidaire d'une tige dont la partie pénétrant dans le cylindre est filetée et traverse un écrou de fermeture de l'extrémité correspondante du corps qui est monté bloqué en translation mais libre en rotation sur cette dernière, et le piston soumis à l'action de moyens élastiques est solidaire d'une tige lisse traversant par un trou lisse un bouchon de fermeture de l'extrémité considérée du cylindre et prenant appui contre un ressort dont l'autre extrémité prend appui sur un épaulement ménagé sur la face interne du cylindre.

Il est donc procédé au réglage axial de la première tige par pivotement de l'écrou que traverse cette tige. Lorsque le réglage souhaité est obtenu, il est procédé au blocage en translation du piston auquel est associée cette tige.

La seconde tige est maintenue poussée élastiquement en position sortie par le ressort. Toutefois, lorsque le piston associé à cette tige n'est pas bloqué en translation, il est possible de déplacer légèrement la tige vers l'intérieur du corps à l'encontre de l'action du ressort. Ceci permet de bénéficier d'un certain jeu élastique.

Selon une forme simple d'exécution de ce dispositif, le corps comporte une cavité de section circulaire délimitant deux chambres coaxiales destinées chacune à recevoir un piston, et débouchant latéralement par une lumière longitudinale, chaque piston servant à la fixation d'une plaquette formant clavette qui, engagée dans une lumière longitudinale, assure le blocage en rotation du piston considéré.

Il est à noter que les clavettes qui assurent le blocage en rotation des pistons permettent également de visualiser la position axiale de ceux-ci, ce qui est particulièrement avantageux notamment pour le premier piston qui est le pistion de réglage. Afin de faciliter la visualisation de la position de ce piston, les bords délimitant la lumière associée au piston réglable axialement comportent une échelle graduée.

Avantageusement, chaque clavette comporte un perçage communiquant avec un perçage diamétral du piston correspondant, ce dernier perçage étant taraudé et servant au montage d'une vis de pression destinée au blocage axial du piston.

Chaque vis de pression étant en position desserrée lors de la mise en place du dispositif, il est procédé après réglage de la première tige au blocage de la vis de pression associée à son piston, et au blocage de la vis de pression associée au second piston si le dispositif doit être utilisé dans le cadre d'un montage rigide.

Enfin, les parties des tiges destinées à recevoir les mâchoires présentent une section polygonale et servent chacune à l'engagement d'une rotule sur laquelle sont montés de façon amovible et réglable deux mors de serrage, dont l'un est équipé de la mâchoire de fixation des vis engagées dans l'os.

De préférence, les mâchoires de fixation des vis engagées dans l'os sont conformées de manière à permettre la fixation d'au moins une des vis non parallèlement aux autres, afin d'augmenter les possibilités de fixation du dispositif en fonction des besoins.

De toute façon, l'invention sera bien comprise à l'aide de la description qui suit, en référence au dessin schématique annexé représentant à titre d'exemple non limitatif, une forme d'exécution de ce dispositif :
Figure 1 en est une vue en perspective en position montée sur la partie inférieure d'une jambe d'un individu ;
Figure 2 en est une vue de face à échelle agrandie ;
Figure 3 en est une vue en coupe longitudinale ;
Figure 4 en est une vue en coupe transversale selon la ligne IV-IV de figure 3.

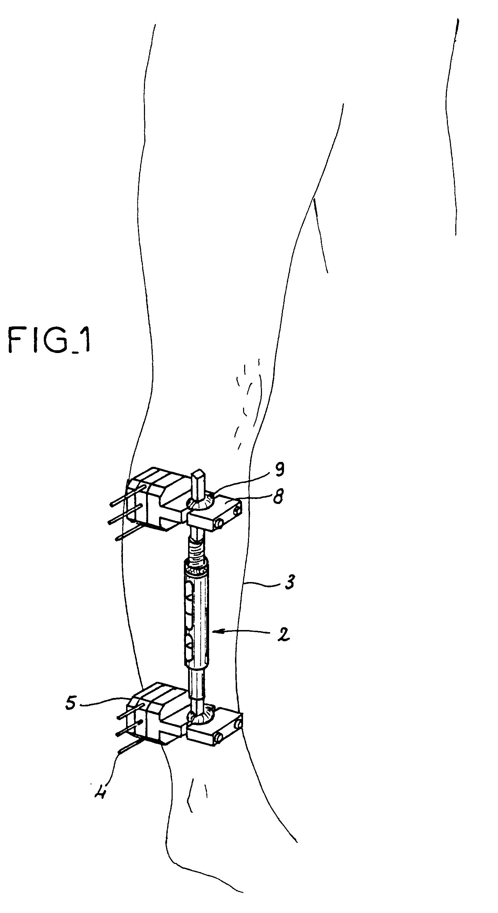

La figure 1 représente le dispositif selon l'invention désigné par la référence générale 2 monté sur la partie inférieure 3 d'une jambe d'un individu. Le dispositif est par exemple utilisé dans ce cas pour réaliser la contention d'un tibia fracture.

Ce dispositif consiste de façon connue en soi à mettre en oeuvre un certain nombre de broches, en l'occurrence trois broches désignées par la référence 4 qui sont vissées dans chacune des deux parties de l'os à réparer. Les broches 4 de chaque série de broches sont tenues par une mâchoire 5. Cette mâchoire 5 est elle-même solidaire d'un mors 6, associé par deux vis 7 à un second mors 8, les mors 6 et 8 étant destines a enserrer de façon réglable une rotule 9. Chaque rotule 9 comporte un perçage central 10 de section sensiblement carrée, de forme complémentaire de celle de l'extrémité d'une tige 12 ou 13.

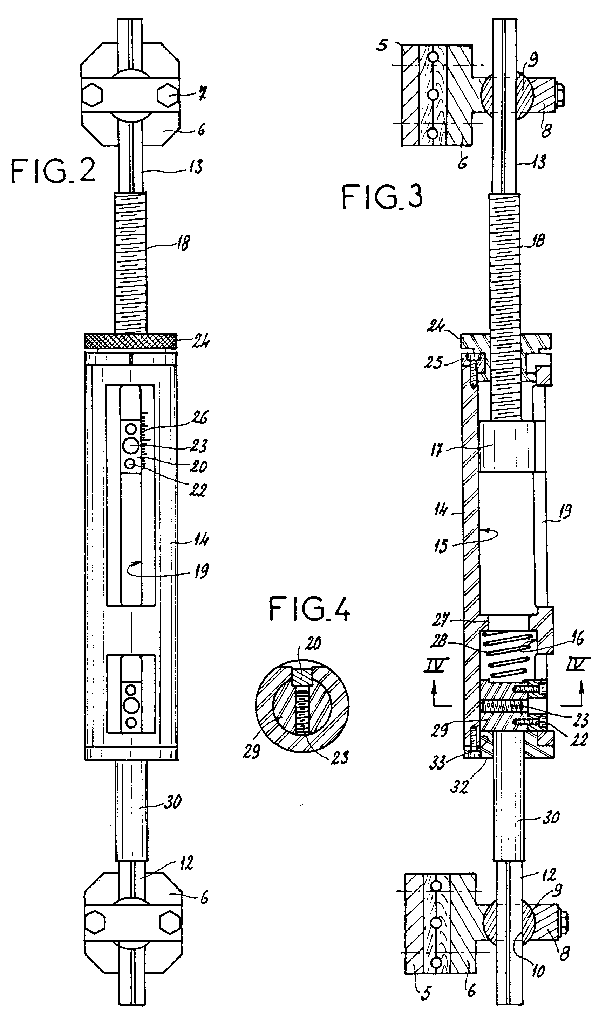

Le dispositif selon l'invention comprend essentiellement un corps 14 en forme de cylindre d'axe sensiblement parallèle au membre à équiper, dans lequel sont ménagées deux chambres coaxiales 15 et 16. La première chambre 15 de section circulaire sert au logement d'un piston 17, également de section circulaire, solidaire d'une tige filetée 18, dont l'extrémité est formée par la tige 13 précitée de section carrée. La chambre 15 communique avec l'extérieur par l'intermédiaire d'une lumière 19 débouchant longitudinalement dans le corps 14. Dans cette lumière 19 est engagée une plaquette 20 formant clavette fixée sur le piston 17 par l'intermédiaire de deux vis 22. En outre, le piston 17 présente un perçage diamétral, non représenté au dessin, disposé dans l'axe d'un perçage de la plaquette 20, servant au logement d'une vis de pression désignée par la référence 23 à la figure 2. Cette vis de pression permet de réaliser le blocage axial du piston 17 dans la position désirée. La tige filetée 18 traverse au niveau de l'extrémité du corps 14, une pièce 24 en forme d'écrou, qui est montée libre en rotation sur le corps, mais immobilisée en translation à l'aide d'une bague 25. Il est donc possible de réaliser le réglage axial de la position du piston 17 par rotation de l'écrou 24. La visualisation de la position du piston est réalisée par l'intermédiaire d'échelles graduées 26 ménagées sur les bords de la fente 19.

La seconde chambre 16 est séparée de la chambre 15 par l'intermédiaire d'un épaulement 27 sur lequel prend appui un ressort 28 dont l'autre extremité prend appui sur un piston 29, solidaire d'une tige lisse 30, dont l'extrémité est constituée par la tige 12 de section carrée. La tige lisse 30 traverse un bouchon de fermeture 32 du corps 14, ce bouchon 32 présentant à cet effet un trou lisse 33.

Le piston 29, de section circulaire, est bloqué en rotation par une plaquette 20 formant clavette, fixée à l'aide de vis 22. Ce piston présente également un alésage diamétral servant au logement d'une vis de pression 23 susceptible de réaliser son blocage axial dans la position souhaitée.

En pratique, les brôches sont tout d'abord mises en place dans les deux tronçons d'os, après quoi elles sont enserrées dans les mâchoires 5 qui sont elles-mêmes montées par l'intermédiaire des mors 6,8 sur les rotules 9 des tiges 12 et 13, après quoi il est procédé au réglage de la position du piston 17 à son blocage axial, et éventuellement au blocage axial du piston 29.

Comme il ressort de ce qui précède, l'invention apporte une grande amélioration à la technique existante, en fournissant un dispositif externe de contention osseuse de structure simple et susceptible d'un grand nombre d'applications tant en position bloquée des deux parties de l'os, que dans un état permettant un certain jeu élastique entre ces deux parties.

Comme il va de soi, l'invention ne se limite pas à la seule forme d'exécution de ce dispositif décrite ci-dessus à titre d'exemple, elle en embrasse au contraire toutes les variantes. C'est ainsi notamment que les moyens de blocage en rotation et en translation des pistons pourraient être différents, ou encore que le montage des mâchoires porte-broches sur les tiges pourrait être différent, sans que l'on sorte pour autant du cadre de l'invention.

## Revendications

1. Dispositif externe de contention osseuse, du type comprenant deux mâchoires (5) destinées chacune à la fixation de plusieurs vis ou similaires engagées dans l'une des deux parties de l'os dont la contention est à réaliser, ces deux mâchoires (5) étant montées sur un même support s'étendant parallèlement au membre à équiper, ce support comprenant un corps (14) en forme de cylindre d'axe sensiblement parallèle au membre à équiper, dans lequel sont montés deux pistons (17,29) dont chacun est solidaire d'une tige destinée à la fixation de l'une des deux mâchoires (5), l'un (17) des deux pistons étant associé à des moyens de réglage de sa position axiale dans le cylindre (14) et à des moyens de blocage dans celui-ci, tandis que l'autre piston (29) est associé à des moyens élastiques (28) tendant à le pousser vers l'extérieur, et à des moyens de blocage dans le cylindre, caractérisé en ce que le piston (17) équipé de moyens de réglage axial est monté bloqué en rotation dans le cylindre mais libre en translation dans celui-ci, et est solidaire d'une tige (13,18) dont la partie (18) pénétrant dans le cylindre est filetée et traverse un écrou (24) de fermeture de l'extrémité correspondante du corps (14), qui est monté bloqué en translation mais libre en rotation sur cette dernière, et en ce que le piston (29) soumis à l'action de moyens élastiques (28) est solidaire d'une tige lisse (30) traversant par un trou lisse un bouchon de fermeture (32) de l'extrémité considérée du cylindre et prenant appui contre un ressort (28) dont l'autre extrémité prend appui sur un épaulement (27) ménagé sur la face interne du cylindre.

2. Dispositif selon la revendication 1, caractérisé en ce que le corps (14) comporte une cavité de section circulaire délimitant deux chambres coaxiales (15,16) destinées chacune à recevoir un piston (17,29), et débouchant latéralement par une lumière longitudinale (19), chaque piston servant à la fixation d'une plaquette (20) formant clavette qui, engagée dans la lumière longitudinale (19), assure le blocage en rotation du piston considéré.

3. Dispositif selon la revendication 2, caractérisé en ce que chaque clavette (20) comporte un perçage communiquant avec un perçage diamétral du piston correspondant, ce dernier perçage étant taraudé et servant au montage d'une vis de pression (23) destinée au blocage axial du piston.

4. Dispositif selon l'une quelconque des revendications 2 et 3, caractérisé en ce que les bords délimitant la lumière (19) associée au piston réglable axialement comportent une échelle graduée (26).

5. Dispositif selon l'une quelconque des revendications 1 à 4, caractérisé en ce que les parties des tiges destinées à recevoir les mâchoires (5) présentent une section polygonale et servent chacune à l'engagement d'une rotule (9) sur laquelle sont montés de façon amovible et réglable deux mors de serrage (6,8), dont l'un est équipé de la mâchoire (5) de fixation des vis engagées dans l'os.

6. Dispositif selon la revendication 5, caractérisé en ce que les mâchoires (5) de fixation des vis dans l'os sont conformées de manière à permettre la fixation d'au moins une des vis non parallèlement aux autres.

## Claims

1. External bone-immobilising device, of the type comprising two jaws (5) each intended for the fixation of several screws or the like engaged in one of the two parts of the bone which is to be immobilised, these two jaws (5) being mounted on the same support extending parallel to the limb to be fitted, this support comprising a body (14) in the shape of a cylinder with its axis substantially parallel to the limb to which it is to be fitted, in which are mounted two pistons (17, 29), each of which is secured to a rod intended for fixing one of the two jaws (5), one (17) of the two pistons being associated with means of adjusting its axial position in the cylinder (14) and with means of locking in the latter, whilst the other piston (29) is associated with elastic means (28) tending to push it outwards, and with means of locking in the cylinder, characterised in that the piston (17) equipped with axial adjustment means is mounted so as to be locked with respect to rotation in the cylinder but free with respect to translation in the latter, and is secured to a rod (13, 18) whose part (18) entering the cylinder is threaded and passes through a nut (24) for closing the corresponding end of the body (14), which is mounted so as to be locked with respect to translation but free with respect to rotation on the latter, and in that the piston (29) subjected to the action of elastic means (28) is secured to a smooth rod (30) passing, by means of a smooth hole, through a plug (32) for closing the relevant end of the cylinder and bearing against a spring (28), the other end of which bears on a shoulder (27) formed on the internal face of the cylinder.

2. Device according to Claim 1, characterised in that the body (14) has a cavity of circular cross section defining two coaxial chambers (15, 16) each intended to receive a piston (17, 29), and opening out laterally through a longitudinal aperture (19), each piston serving to fix a plate (20) forming a key which, engaged in the longitudinal aperture (19), provides the locking of the relevant piston with respect to rotation.

3. Device according to Claim 2, characterised in that each key (20) has a drilling communicating with a diametral drilling in the corresponding piston, the latter drilling being threaded and being used for the mounting of a locking screw (23) intended for the axial locking of the piston.

4. Device according to either one of Claims 2 and 3, characterised in that the edges defining the aperture (19) associated with the axially adjustable piston have a graduated scale (26).

5. Device according to any one of Claims 1 to 4, characterised in that the parts of the rods intended to receive the jaws (5) have a polygonal cross section and are each used for the engagement of a pivot (9) on which are mounted, removably and adjustably, two clamping vices (6, 8), one of which is equipped with the jaw (5) for fixation of the screws engaged in the bone.

6. Device according to Claim 5, characterised in that the jaws (5) for fixation of the screws in the bone are shaped so as to enable at least one of the screws to be fixed other than parallel to the others.

## Patentansprüche

1. Externe Knochenfixierungsvorrichtung des Typs, umfassend zwei Klemmeinrichtungen (5), welche jeweils zur Befestigung mehrerer Schrauben oder dgl. bestimmt sind, welche in einen der beiden Teile des zu fixierenden Knochens eingreifen, wobei die beiden Klemmeinrichtungen (5) auf einem gleichen, sich parallel zu dem auszustattenden Glied erstreckenden Träger angebracht sind, welcher einen zylinderförmigen Körper (14) mit zu dem auszustattenden Glied im wesentlichen paralleler Achse umfaßt, in welchem zwei Kolben (17, 29) angebracht sind, von denen jeder mit einer zur Befestigung einer der beiden Klemmeinrichtungen (5) bestimmten Stange verbunden ist, wobei einem (17) der beiden Kolben Mittel zum Einstellen seiner Axialposition in dem Zylinder (14) und Mittel zum Feststellen in diesem zugeordnet sind, während dem anderen Kolben (29) elastische, ihn nach außen drängende Mittel (28) und Mittel zum Feststellen in dem Zylinder zugeordnet sind, dadurch gekennzeichnet, daß der mit Mitteln zur Axialeinstellung ausgestattete Kolben (17) unverdrehbar, aber verschiebbar in dem Zylinder angebracht ist und mit einer Stange (13, 18) verbunden ist, deren in den Zylinder eindringender Abschnitt (18) ein Gewinde aufweist und eine das entsprechende Ende des Körpers (14) verschließende Schraubenmutter (24) durchsetzt, welche unverschiebbar aber drehbar an letzterem angebracht ist, und daß der der Wirkung der elastischen Mittel (28) ausgesetzte Kolben (29) mit einer glatten Stange (30) verbunden ist, welche durch ein glattes Loch einen das betreffende Ende des Zylinders (14) verschließenden Deckel (32) durchsetzt, und sich an einer Feder (28) abstützt, deren anderes Ende an einer Schulter (27) abgestützt ist, welche an der Innenfläche des Zylinders ausgebildet ist.

2. Vorrichtung nach Anspruch 1, dadurch gekennzeichnet, daß der Körper (14) einen Hohlraum kreisförmigen Querschnitts umfaßt, welcher zwei koaxiale, jeweils zur Aufnahme eines Kolbens (17, 29) bestimmte Kammern (15, 16) festlegt und zur Seite hin in einen Längsschlitz (19) mündet, wobei jeder Kolben zur Befestigung eines ein Blockierteil bildenden Plattenelements (20) dient, welches durch Eingriff in den Längsschlitz (19) die Unverdrehbarkeit des betreffenden Kolbens gewährleistet.

3. Vorrichtung nach Anspruch 2, dadurch gekennzeichnet, daß jedes Blockierteil (20) eine Bohrung aufweist, welche mit einer diametralen Bohrung des entsprechenden Kolbens in Verbindung steht, wobei diese letztere Bohrung ein Innengewinde aufweist und zur Befestigung einer Klemmschraube (23) dient, welche zur axialen Feststellung des Kolbens bestimmt ist.

4. Vorrichtung nach einem der Ansprüche 2 und 3, dadurch gekennzeichnet, daß die Ränder des dem axial einstellbaren Kolben zugeordneten Schlitzes (19) eine mit Gradteilung versehene Skala (26) umfassen.

5. Vorrichtung nach einem der Ansprüche 1 bis 4, dadurch gekennzeichnet, daß die zum Aufnehmen der Klemmeinrichtungen (5) bestimmten Abschnitte der Stangen einen polygonalen Querschnitt aufweisen und jeweils zum Eingriff in ein Kugelgelenk (9) dienen, an welchem in entfernbarer und einstellbarer Weise zwei Spannbacken (6, 8) angebracht sind, von denen die eine mit der Klemmeinrichtung (5) zur Befestigung der in den Knochen eingreifenden Schrauben ausgestattet ist.

6. Vorrichtung nach Anspruch 5, dadurch gekennzeichnet, daß die Klemmeinrichtungen (5) zur Befestigung der Schrauben in dem Knochen derart angepaßt sind, daß sie die nicht parallele Befestigung wenigstens einer der Schrauben relativ zu den anderen ermöglichen.
